# EUROPEAN PATENT SPECIFICATION

(11) **EP 0 577 147 B1**
(45) Date of publication and mention of the grant of the patent: **23.09.1998**
(21) Application number: 93111742.8
(22) Date of filing: 20.06.1989
(51) Int. Cl.: A23L 1/236, A23L 1/222

(54) **Method for stabilizing taste-modifier**
Verfahren zum Stabilisieren eines Geschmacksveränderungsmittels
Procédé de stabilisation d'un agent modifiant le goût

(30) Priority: 21.06.1988 JP 153143/88; 02.11.1988 JP 277717/88; 11.11.1988 JP 285473/88
(43) Date of publication of application: 05.01.1994
(62) Divisional of application: 89111181.7
(73) Proprietor: Kurihara, Yoshie, Tokyo 125 (JP); ASAHI DENKA KOGYO KABUSHIKI KAISHA, Arakawa-ku Tokyo 116 (JP)
(72) Inventor: Kurihara, Yoshie, Tokyo 125 (JP); Kohno, Hiroshige, c/o ASAHI DENKA KOGYO K.K., Tokyo 116 (JP); Kato, Masaaki, c/o ASAHI DENKA KOGYO K.K., Tokyo 116 (JP); Ikeda, Kenji, c/o ASAHI DENKA KOGYO K.K., Tokyo 116 (JP); Miyake, Masako, c/o ASAHI DENKA KOGYO K.K., Tokyo 116 (JP)
(74) Representative: Grünecker, Kinkeldey, Stockmair & Schwanhäusser Anwaltssozietät

(56) References cited:
- E.SPREER 'Technologie der Milchverarbeitung' 1974 , VEB , LEIPZIG pages 91,92 *4.2.5.2.Erhitrungverfahren*

## Description

This invention relates to a method for stabilizing a taste-modifier which comprises peeled fresh Curculigo latifolia fruits, dried fruits thereof or a curculin-containing material obtained therefrom.

### (Description of the Prior Art)

Known taste-modifiers which affect the receptor membranes on the tongue in such a manner as to modify the taste of a food, include those which remove the sweetness of a sweet food in the mouth, for example, gymnemic acid contained in Gymnema sylvestre leaves and ziziphine contained in Ziziphus jujuba leaves; and those which convert the sourness of a sour food into sweetness in the mouth, for example, miraculin contained in Synsepulm dulcificum fruits.

Although miraculin has the abovementioned effect, it is not put into practical use as a taste-modifier because of its poor stability.

The present inventors have found that a sour material or water taken after eating Curculigo latifolia fruits would taste sweet. Thus they have attempted to identify the sweetness-inducer. As a result, they have found that a specific protein contained in Curculigo latifolia fruits is the aimed sweetness-inducer (cf. Japanese Patent Application No. 153143/1988). This protein is named curculin. In order to utilize this curculin as a taste-modifier on a commercial scale, it is required to obtain crude curculin in a stable form from Curculigo latifolia fruits as efficiently as possible.

Pure curculin may be obtained by washing fresh Curculigo latifolia fruits or dried fruits thereof with water, extracting from them with an aqueous solution of a salt and purifying the extract by ion-exchange chromatography with the use of CM-Sepharose and HPLC with the use of a gel column. Pure or almost pure curculin would remain stable for a month or longer in the form of an aqueous solution at room temperature. However , fresh Curculigo latifolia fruits, dried fruits thereof or crude curculin, in particular, in the form of an aqueous solution, would have a poor stability upon storage since they are contaminated with proteases and bacteria contained in the Curculigo latifolia fruits.

From E. Speer "Technologie der Milchverarbeitung", 3rd edition, 1974, pages 355, 356, 91 VEB Fachbuchverlag, Leipzig, DE, a process for pasteurising milk by heating to 62-65°C for 30 minutes; 68-72°C for 8 to 40 seconds; 71 to 74°C for 40 to 45 seconds; or 85°C for 8 to 15 seconds is known. The object is to eliminate or reduce the bacterial count of the milk.

US Patent No. 4,587,132 describes a process for preparing a fruit puree product made from natural fruits. It is mentioned that the fruit product is added to boiling water containing organic acids and then heated to 200°F, i.e. 93.3°C. It is furthermore suggested to treat the fruit product at the minimum permissible temperature that will deactivate enzymes and destroy certain micro-organisms and to create an environment which will not support microbial growth. From this prior art, no solution for stabilising a taste-modifier was known.

### SUMMARY OF THE INVENTION

It is an object of the present invention to provide a method for stabilizing a taste-modifier comprising a protein curculin in a state of a crude product or an aqueous solution for a prolonged period of time.

The present inventors have conducted extensive studies in order to achieve the above object. As a result, they have found that the protein curculin is stable to heat and thus the taste-modification activity thereof would be never lowered when Curculigo latifolia fruits are heated in order to pasteurize the same as well as to inactivate proteases contained therein.

Accordingly the present invention, which has been completed based on the above finding, provides the following method (1) for stabilizing a taste-modifier.
(1) A method for stabilizing a taste-modifier comprising peeled fresh Curculigo latifolia fruits, dried fruits thereof or a curculin-containing material obtained therefrom which comprises heating said taste-modifier to 50 to 90 °C for 5 min to 24 hr.

According to the method for stabilizing a taste-modifier of the present invention, a taste-modifier comprising peeled fresh Curculigo latifolia fruits, dried fruits thereof or a curculin-containing material obtained therefrom can be heated and proteases contained therein can be inactivated without lowering the taste-modification activity of the same. Thus the taste-modifier can be stably preserved for a prolonged period of time in the form of a crude product or an aqueous solution.

### DETAILED DESCRIPTION OF THE INVENTION

Now the invention will be described.

The taste-modifier comprising peeled fresh Curculigo latifolia fruits, dried fruits thereof or a curculin-containing material obtained therefrom can be stabilized by heating to 50 to 90 °c, preferably 60 to 80°C for 5 min. to 24 hr. Thus it can be pasteurized and proteases contained therein can be simultaneously inactivated.

The necessary heating period would increase with a decrease in the heating temperature. For example, it is preferable to heat the taste-modifier to 50°C for 30 minutes or longer, preferably 30 minutes to 24 hours; and to 90°c for five minutes or longer, preferably 5 to 60 minutes.

When the heating temperature is lower than 50°c, the aimed pasteurization and inactivation effects can not be fully achieved. When it exceeds 90°C, on the other hand, the taste-modification effect of the taste-modifier might be lowered.

After the completion of the heating, the taste-modifier may be filled in a container or dried. It may be stabilized and dried with a hot air stream at 50 to 90°C.

According to the present invention, the taste-modifier comprising peeled fresh Curculigo latifolia fruits, dried fruits thereof or a curculin-containing material obtained therefrom is heated to 50 to 90°C for 5 min. to 24 hr. to thereby stabilize said taste-modifier.

The method (1) for stabilizing the taste-modifier of the present invention may be conducted at any stage in the purification of curculin. In addition, these methods may be applied to the taste-modifier in and form, i.e., a dried matter, a solution or dispersion in water or a product containing the same.

The fresh Curculigo latifolia fruits or dried fruits thereof constituting the taste-modifier to be used in the present invention shall be free from peels and seeds, since no curculin is contained in these parts.

The method for drying Curculigo latifolia fruits is not particularly restricted. Namely, sun-dried Curculigo latifolia fruits, hot air-dried ones and lyophilized ones such as lyophilized pulp may be used in the present invention.

The peeled fresh Curculigo latifolia fruits or dried fruits thereof may be generally ground, milled or pasted prior to the use, though the form of the optionally dried curculigo latifolia fruits is not particularly restricted.

Examples of the curculin-containing material obtained from peeled fresh Curculigo latifolia fruits or dried fruits thereof described above include curculin extracted from fresh Curculigo latifolia fruits, dried fruits thereof or the residue obtained by appropriately treating the fresh Curculigo latifolia fruits or dried fruits thereof and removing a curculin-free component therefrom. The concentration of the curculin extracted from fresh Curculigo latifolia fruits or dried fruits thereof is not particularly restricted. Namely, either a highly pure curculin or an extract containing a large amount of materials other than the curculin may be used in the present invention. Further the extract may be mixed with other components.

The extraction of the curculin is not particularly restricted. A preferable example thereof comprises extracting from peeled fresh Curculigo latifolia fruits or dried fruits thereof with an aqueous solution of a salt at a concentration of at least 0.01 M. Examples of the salt include chlorides such as sodium chloride, potassium chloride, calcium chloride, magnesium chloride and ammonium chloride; phosphates such as sodium phosphate, potassium phosphate, magnesium phosphate and ammonium phosphate; carbonates such as sodium carbonate, potassium carbonate, magnesium carbonate and ammonium carbonate; sulfates such as sodium sulfates, magnesium sulfate, calcium sulfate and ammonium sulfate; sulfites such as sodium sulfite, magnesium sulfite, calcium sulfite and ammonium sulfite; nitrates such as sodium nitrate and potassium nitrate; nitrites such as sodium nitrite and potassium nitrite; lactates such as sodium lactate and calcium lactate; alum; burnt alum; sodium acetate; pyrophosphates such as sodium pyrophosphate and potassium pyrophosphate; propionates such as sodium propionate and calcium propionate; sodium benzoate; sodium fumarate; and sodium polyacrylate.

A typical example of the extraction of curculin with the aqueous solution of a salt may be carried in the following manner.

An aqueous solution of a salt such as sodium chloride is added to fresh Curculigo latifolia fruits or dried fruits thereof and the obtained mixture is homogenized followed by filtering and centrifuging. Since curculin is contained in the water-insoluble part of Curculigo latifolia sarcocarp, it is preferable to homogenize the above mixture of the fresh Curculigo latifolia fruits or dried fruits thereof and water followed by thoroughly washing the mixture to thereby remove the water-soluble part and extracting from the residue with the above-mentioned salt solution so as to elevate the purity of curculin.

The concentration of the salt of the aqueous solution to be used for the extraction should exceed 0.01 M, since curculin can not be sufficiently extracted with a salt solution of a concentration lower than 0.01 M. On the other hand, a salt solution of an excessively high concentration requires a prolonged period of time for desalting following the extraction. Thus the concentration of the salt solution preferably ranges from 0.1 to 1.0 M, from the viewpoints of the extraction efficiency and the subsequent purification procedure.

The extract thus obtained with the use of the salt solution is then desalted and dried to thereby give a curculin-containing material which is sufficiently available in practice. However the purity of curculin can be further elevated by purifying the above extract by ion exchange chromatography with the use of CM-Sepharose and HPLC with the use of a gel column followed by desalting and drying. Thus pure curculin can be obtained. It is a matter of course that the curculin purity may be further elevated by various purification procedures other than those described above, for example, known protein purification procedures such as salting-out or solvent precipitation.

A typical example of the curculin thus obtained is a protein having a molecular weight of approximately 12,500 dalton, an amino acid residue number of 97 and an iso-electric point of 7.1. This protein is present as a dimer of a molecular weight of approximately 26,000 dalton. The following Table 1 shows the amino acid composition of this protein. Thus it contains relatively large amounts of aspartic acid, leucine and glycine.

**Table 1**

| Amino acid composition | | |
|---|---|---|
| Amino acid | % by mol | No. of residues |
| Aspartic acid (Asp) | 17.3 | 17 |
| Threonine (Thr) | 6.4 | 6 |
| Serine (Ser) | 7.0 | 7 |
| Glutamic acid (Glu) | 7.2 | 7 |
| Proline (Pro) | 1.2 | 1 |
| Glycine (Gly) | 12.5 | 12 |
| Alanine (Ala) | 5.3 | 5 |
| Cystine (Half-cys) | - | - |
| Valine (Val) | 6.8 | 7 |
| Methionine (Met) | 0.4 | 1 |
| Isoleucine (Ile) | 4.2 | 4 |
| Leucine (Leu) | 14.5 | 14 |
| Tyrosine (Tyr) | 5.2 | 5 |
| Phenylalanine (Phe) | 1.3 | 1 |
| Lysine (Lys) | 2.7 | 3 |
| Histidine (His) | 2.4 | 2 |
| Arginine (Arg) | 5.5 | 5 |
| Total | | 97 |

To further illustrate the present invention, the following Examples will be given.

### Example 1

Curculigo latifolia fruits were washed with water and peels and seeds were removed therefrom. The residual sarcocarp was ground in a mortar.

Then the ground sarcocarp was dried with a hot air stream at 65°C for 24 hours to thereby give a taste-modifier.

100 mg of the taste-modifier was kept in the mouth for one minutes and then vomitted. Subsequently a 0.02 M aqueous solution of citric acid was kept in the mouth. Then the citric acid solution tasted sweet just like a sugar solution does.

This taste-modifier was introduced into an sterile Petri dish and allowed to stand at 37°C for three months. Then it was subjected to the same sensory test as the one described above. As a result, it showed an intense sweetness similar to the above case.

### Example 2

50 ml of water was added to 16 g of Curculigo latifolia sarcocarp ground in the same manner as the one described above. Then the mixture was homogenized and centrifuged at 10,000 rpm for 30 minutes. After removing the supernatant, 50 ml of water was added to the resuide and the obtained mixture was homogenized. Then it was heated to 70°C for 20 minutes and centrifuged at 10,000 rpm for 30 minutes. After removing the supernatant, the residue was obtained.

This residue was dried under reduced pressure to thereby give a taste-modifier.

10 mg of this taste-modifier was kept in the mouth for a minutes and then vomitted. Subsequently a 0.02 M aqueous solution of citric acid was taken. As a result, the citric acid solution showed an intense sweetness just like a sugar solution does.

This taste-modifier was introduced into a sterile Petri dish and allowed to stand at 37°C for three months. Then it was subjected to the same sensory test as the one described above. As a result, it showed an intense sweetness similar to the above case.

### Comparative Example 1

Curculigo latifolia sarcocarp ground in the same manner as the one described in Example 1 was lyophilized to thereby give a taste-modifier.

Immediately after the preparation, this taste-modifier showed a taste-modification effect comparable to that of the taste-modifier of Example 1. After allowing to stand in a sterile Petri dish for three months at 37°C, however, it showed no sweetness but a sourness in the same sensory test as the one described above.

### Comparative Example 2

The procedure of Example 2 was repeated except that no heating was conducted to thereby give a taste-modifier.

This taste-modifier was allowed to stand in a sterile Petri dish for three months at 37°C and then subjected to the same sensory test as the one described in Example 2. As a result, it showed a slight sweetness and an intense sourness.

## Claims

1. A method for stabilizing a taste-modifier comprising peeled fresh Curculigo latifolia fruits, dried fruits thereof or a curculin-containing material obtained therefrom, which comprises heating said taste modifier to 50-90°C for 5 min to 24 hr.

## Patentansprüche

1. Ein Verfahren zum Stabilisieren eines Geschmacksverwandlers, umfassend geschälte, frische oder getrocknete Curculigo latifolia-Früchte oder ein davon erhaltenes Curculin-enthaltendes Material, umfassend Erhitzen dieses Geschmacksverwandlers auf 50 bis 90°C für 5 Minuten bis 24 Stunden.

## Revendications

1. Procédé pour stabiliser un agent modifiant le goût comprenant des fruits de Curculigo latifolia frais pelés, leurs fruits séchés ou une matière contenant de la curculine obtenue à partir de ceux-ci, qui comprend le chauffage dudit agent modifiant le goût à 50 à 90°C pendant 5 min à 24 h.
